# EUROPEAN PATENT SPECIFICATION

(11) **EP 2 849 583 B1**
(45) Date of publication and mention of the grant of the patent: **13.04.2016**
(21) Application number: 13723063.7
(22) Date of filing: 08.05.2013
(51) Int. Cl.: A23L 5/43, A23L 2/58, A61K 47/12, A61K 47/46

(54) **A DISPERSION PRODUCT COMPRISING NORBIXIN AS AN ORANGE/RED PIGMENT**
DISPERSIONSPRODUKT MIT NORBIXIN ALS ORANGES/ROTES PIGMENT
PRODUIT DE DISPERSION COMPORTANT DE LA NORBIXINE EN TANT QUE PIGMENT ROUGE/ORANGE

(30) Priority: 16.05.2012 EP 12168161
(43) Date of publication of application: 25.03.2015
(73) Proprietor: Chr. Hansen Natural Colors A/S, 2970 Hørsholm (DK)
(72) Inventor: KOEHLER, Klaus, DK-1820 Frederiksberg (DK); NIELSEN, Lene Munch, DK-2700 Broenshoej (DK)
(86) International application number: PCT/EP2013/059575
(87) International publication number: WO 2013/171108

(56) References cited:
- WO-A1-2011/119290
- WO-A1-2011/154407
- WO-A2-2008/128766
- DATABASE WPI Week 199522 Thomson Scientific, London, GB; AN 1995-167396 XP002685710, & JP 7 090188 A (SANEIGEN FFI KK) 4 April 1995 (1995-04-04)
- DATABASE WPI Week 201224 Thomson Scientific, London, GB; AN 2012-C88682 XP002685709, & JP 2012 046573 A (TOYO INK MFG CO LTD) 8 March 2012 (2012-03-08)

## Description

### FIELD OF THE INVENTION

The present invention relates to an orange/red coloring composition comprising norbixin as an orange/red pigment. It may be used in the manufacture of food and pharmaceutical products.

### BACKGROUND ART

Norbixin is today used commercially as an orange/red pigment in different food products.

The commercially available acid stable (e.g. in soft drink) transparent natural red color product of Chr. Hansen A/S, Denmark termed "A-720-WS-AP" comprises following ingredients: norbixin (E 160b), propylene glycol (E 1520) (48%), polysorbate 80 (E 433) (48%) - see e.g. the publicly available product information.

When this natural red A-720-WS-AP product is used in e.g. soft drinks (low pH around 3-4) a stable transparent orange/red color is obtained.

It is transparent due to the fact that the norbixin (orange/red color) particles are very small in the suspension - i.e. very small norbixin (orange/red color) particles dispersed in the low pH water phase (e.g. soft drink).

One may term this a solid-in-water dispersion - which, as known to the skilled person, is different from e.g. an oil-in-water emulsion.

As known in the art Polysorbate 80 (Tween 80) is a detergent/emulsifier - herein alternatively expressed dispersing agent.

As known in the art Polysorbate 80 (Tween 80) may be said to have an unwanted unpleasant taste.

Another natural orange/red color product of Chr. Hansen A/S, Denmark is termed CapColors®A-8-WSS-145 and it may be characterized as made by milling in order to create sufficiently small bixin (orange/red color) particles. According to the public available product information - for this product the color will look more or less opaque (i.e. one may say that it is less transparent than the A-720-WS-AP product discussed above).

The CapColors®A-8-WSS-145 product does not comprise Polysorbate 80 and may therefore be seen as a prior art solution to make a product without the unpleasant taste of Polysorbate 80 (Tween 80).

The compound quillaja is a known detergent/emulsifier.

WO2011/119290A1 and WO2011/154407A1 describe use of quillaja as emulsifier in an oil-in-water emulsion.

Claim 14 of WO2011/154407A1 relates to an oil-in-water emulsion comprising bixin.

As known in the art - bixin is soluble in oil/fat and not in water at any herein relevant pH range.

As known in the art, the herein relevant norbixin orange/red pigment is not soluble in oil/fat.

The two WO documents do not describe use of quillaja in a herein relevant solid-in-water dispersion (such as e.g. a soft drink).

WO2008/128766 describes a beverage containing A-720-WS-AP.

### SUMMARY OF THE INVENTION

The problem to be solved by the present invention is to provide an orange/red coloring composition, giving an orange/red transparent color in acid applications, comprising norbixin as an orange/red pigment, wherein the orange/red coloring composition does not have a herein relevant unwanted unpleasant taste (i.e. is not comprising significant amounts of Polysorbate 80 (Tween 80)).

The solution is based on the fact that the present inventors has identified that a water-dispersible composition using quillaja as dispergent agent is very good in relation to use of norbixin as an orange/red pigment - see working examples herein for further details.

An advantage of quillaja is that it does not have a herein relevant unwanted unpleasant taste (i.e. like Polysorbate 80 (Tween 80)).

As can be seen in reference example 3 herein - for instance polyglycerol is NOT working satisfactory for norbixin - i.e. one may say that quillaja works surprisingly better.

Polyglycerol and quillaja have both a relatively high Hydrophilic-lipophilic balance (HLB) value - however, even though Polyglycerol has a relatively high HLB value it is not working properly in the present context.

Disclosed herein is a water-dispersible red composition comprising from 0.5% to 70% (w/w on a water free basis) of norbixin as an orange/red pigment and from 1.0% to 80% (w/w on a water free basis) of quillaja and wherein the composition comprises less than 90% (w/w) of water.

An example of this is shown in working Example 1 herein - wherein the composition comprises approximately 5% (w/w on a water free basis) of norbixin and approximately 9% (w/w on a water free basis) of quillaja.

The rest of the material/substance of the composition/suspension of Example 1 herein was Propylene glycol: approximately 85% (w/w on a water free basis), around 28-30% of water and some relatively minor amounts of KOH and maybe some methanol from the hydrolysis of bixin.

As known in the art - norbixin is soluble in water at alkaline pH and relatively insoluble at lower pH ranges - such as at pH around pH 3, which is roughly the pH value of e.g. commercially relevant soft drink products.

The water-dispersible composition as described herein may advantageously be used for orange/red coloring of a herein relevant edible, drinkable or pharmaceutical product - such as in particular a product with a herein relevant not alkaline pH value.

By using water-dispersible composition as described herein for orange/red coloring it is possible to obtain a stable transparent orange/red solid-in-water dispersion product (e.g. a soft drink or a wine gum product) - see e.g. working Example 2 herein, where a stable transparent orange/red soft drink product was made.

Accordingly, the invention relates to a transparent orange/red solid-in-water dispersion product comprising norbixin particles as an orange/red pigment and wherein the pH of the dispersion is from pH 1 to pH 8, characterised in that it contains quillaja as a dispersant agent and wherein the ratio (w/w) of norbixin and quillaja is a ratio from 0.01 to 10.

A method for preparing the water-dispersible composition is also disclosed herein, wherein the method comprises the following steps:
(i): preparing a solution of an adequate amount of norbixin in a solvent;
(ii): adding the solution of step (i) to a solution of quillaja under stirring to thereby obtain the water-dispersible composition.

A method for making a transparent orange/red solid-in-water dispersion product of the invention is also described herein, wherein the method comprises dispersing the water-dispersible composition of the first aspect into a water based product with a pH 1 to pH 8 to thereby obtain the transparent orange/red solid-in-water dispersion product of the invention.

Also disclosed herein is the use of the water-dispersible composition as described herein to orange/red coloring of an edible, a drinkable or a pharmaceutical product.

### DEFINITIONS

All definitions of herein relevant terms are in accordance of what would be understood by the skilled person in relation to the herein relevant technical context.

The term "pigment" relates to a material that changes the color of light it reflects as the result of selective color absorption. This physical process differs from fluorescence, phosphorescence, and other forms of luminescence, in which the material itself emits light.

The term "orange/red pigment" or "red pigment" simply relates to a pigment that gives an orange/red color. When used as a pigment as described herein norbixin will always give an orange/red color and the term "orange/red" in relation to use of norbixin as described herein may therefore be seen as merely descriptive - i.e. not a limiting term. As known in the art - the orange color is obtained when a product (e.g. a soft drink) comprises a relatively low concentration of norbixin and a red color is obtained when a product (e.g. a soft drink) comprises a relatively higher concentration of norbixin. A highly concentrated product/composition may be red brown, which is still understood as red in the present context.

The term "orange/red coloring" in relation to the use of the water-dispersible composition as described herein simply relates to the fact that the norbixin pigment gives an orange/red color.

The term "solid-in-water dispersion" should be understood as the skilled person would understand it in the present context. For instance, in relation to a transparent orange/red solid-in-water dispersion product as discussed herein - norbixin particles are solid particles dispersed in the water phase of the solid-in-water dispersion.

Embodiment of the present invention is described below, by way of examples only.

### DETAILED DESCRIPTION OF THE INVENTION

### Water-dispersible composition

Beside the norbixin (as an orange/red pigment) and the quillaja the water-dispersible composition as described herein may of course comprise other herein relevant materials/substances.

For instance the water-dispersible composition may comprise another emulsifier (dispersant agent) than quillaja.

As evident to the skilled person, when there herein is described relevant percentage (%) of e.g. norbixin (as an orange/red pigment) and e.g. quillaja the sum of such percentages can of course not exceed 100% in the water-dispersible composition as described herein.

Norbixin is as such a well known orange/red pigment - it has the common E-number E160b (see EU food additive legislation).

As known - this E-number E160b relates to both norbixin and bixin.

As known in the art - bixin is an apocarotenoid found e.g. in annatto, a natural food coloring obtained from the seeds of the achiote tree (Bixa orellana). Annatto seeds contain about 5% pigments, which consist of approximately 70-80% bixin.

Bixin is soluble in fats but insoluble in water. Upon exposure to alkali, the methyl ester is hydrolyzed to produce the dicarboxylic acid norbixin, a watersoluble derivative.

Without being limited to theory - it may be that norbixin is partly or completely esterified when e.g. an alcohol such as e.g. propylene glycol is used as solvent as described herein (see e.g. working example 1).

Accordingly, the term norbixin includes herein also partly or completely esterified norbixin.

Quillaja is as such known to the skilled person.

An example of a herein suitable quillaja product is a to 20% in water diluted quillaja product that has the common E-number E999 (see EU food additive legislation)

In working Example 1 herein the commercially available product Q-Naturale® from National Starch, UK was used.

The Merck Index, Twelfth Edition mentions examples of herein suitable quillaja product/sources - such as e.g. Merck Index number 8222 (Quillaja, from bark) and Merck Index 8223 (Quillaja, Saponin).

In the present context it may be preferred to have a relative concentrated water-dispersible red composition as described herein - e.g. in order to have a concentrated color product, wherein one e.g. only has to add a few drops to e.g. a soft drink product in order to get a soft drink with a herein relevant suitable orange/red color.

Accordingly, it may be preferred that the water-dispersible red composition as described herein comprises less than 70% (w/w) of water, more preferably less than 50% (w/w) of water and even more preferably less than 40% (w/w) of water.

Preferably, the water-dispersible composition comprises from 2% to 95% (w/w on a water free basis) of a solvent, such as e.g. propylene glycol as a solvent.

In working Example 1 herein is described a preferred composition comprising approximately 85% propylene glycol (w/w on a water free basis) as a solvent.

Preferably, the water-dispersible composition comprises from 20% to 90% (w/w on a water free basis) of a solvent, such as e.g. propylene glycol as a solvent.

The water-dispersible composition may be a liquid composition, in particular wherein the liquid composition is a liquid solution.

The composition of working Example 1 herein is an example of a liquid solution.

Alternatively, the water-dispersible composition may be a dry powder composition.

Preferably, the water-dispersible red composition as described herein comprising from 1% to 40% (w/w on a water free basis) of norbixin as an orange/red pigment and from 2% to 60% (w/w on a water free basis) of quillaja; more preferably the water-dispersible red composition as described herein comprising from 1% to 15% (w/w on a water free basis) of norbixin as a orange/red pigment and from 3% to 20% (w/w on a water free basis) of quillaja.

### A transparent orange/red solid-in-water dispersion

As discussed above - an example of a herein relevant transparent orange/red solid-in-water dispersion of the second aspect could e.g. be a soft drink or wine gum.

As discussed above - a herein relevant advantage of water-dispersible composition as described herein it that it may be used obtain a stable transparent orange/red solid-in-water dispersion product (e.g. a soft drink or a wine gum product) - see e.g. working Example 2 herein, where a stable transparent orange/red soft drink product was made.

Accordingly, as discussed above-the invention relates to a transparent orange/red solid-in-water dispersion product comprising norbixin particles as a orange/red pigment and wherein the pH of the dispersion is from pH 1 to pH 8, characterised in that it contains quillaja as a dispersant agent and wherein the ratio (w/w) of norbixin and quillaja is a ratio from 0.01 to 10.

Without being limited to theory - it is believed that quillaja works as dispersant agent in the sense that it stably disperses the norbixin particles so there is not unwanted sedimentation of the norbixin particles.

As known to the skilled person - when there is unwanted sedimentation of the norbixin particles the herein relevant orange/red color will be lost.

Further - it is believed that quillaja works as a dispersant agent in the sense that it is involved in getting norbixin particles of a relatively small size.

As known to the skilled person - if the norbixin particles are too big in size then the product (e.g. a soft drink) will not be properly transparent.

A further advantage of such small particles is that they have very little tendency of sedimentation.

In the present context - the skilled person knows whether a herein relevant product is properly transparent or not and this will generally depend on the specific product of interest (e.g. if it is e.g. a soft drink or e.g. wine gum).

The term "transparent" of the second aspect should be understood in this context - i.e. as the skilled person will understand it in the present context.

As discussed above - the transparency is related to the size of the norbixin particles and this is related to the ratio (w/w) of norbixin and quillaja in the transparent product of interest.

As evident to the skilled person in the present context - if there is an indefinitely small amount of quillaja present as compared to the amount of norbixin then the effect of the quillaja will be very limited and one would then get e.g. too largely sized norbixin particles (i.e. bad transparency) or sedimentation of the norbixin (i.e. orange/red color is lost).

Preferably, the transparent orange/red solid-in-water dispersion as described herein is a dispersion, wherein the ratio (w/w) of norbixin and quillaja is a ratio from 0.03 to 5; more preferably a ratio from 0.05 to 2 and even more preferably a ratio from 0.1 to 1.

Economically quillaja may be seen as a production cost herein (it is not as such giving the orange/red color).

Accordingly, overall one may say that it is herein preferred to use a minimum amount of quillaja - but still enough to get the herein relevant stable transparent orange/red color.

Preferably, the transparent orange/red solid-in-water dispersion as described herein is a dispersion, wherein the norbixin particles are having an average particle diameter of less than 5 µm, more preferably the norbixin particles are having an average particle diameter of less than 2 µm, even more preferably the norbixin particles are having an average particle diameter of less than 1 µm and most preferably the norbixin particles are having an average particle diameter of less than 200 nm.

A transparent orange/red solid-in-water dispersion as described herein may be a dispersion, wherein the pH of the dispersion is from pH 1 to pH 6 or wherein the pH of the dispersion is from pH 1 to pH 5.

As discussed above - the pH of a soft drink may be around pH 3.

A transparent orange/red solid-in-water dispersion may be a herein relevant edible, drinkable or pharmaceutical product.

Preferably, transparent orange/red solid-in-water dispersion is a food product or a beverage product - preferably for human use.

Preferred examples of edible or drinkable product may be a soft drink, wine gum, marmalade or jam.

### A method for preparing the water-dispersible composition

As discussed above, a method for preparing the water-dispersible composition is described, wherein the method comprises the following steps:
(i): preparing a solution of an adequate amount of norbixin in a solvent; and
(ii): adding the solution of step (i) to a solution of quillaja under stirring to thereby obtain the water-dispersible composition.

As evident to the skilled person one may make adequate adjustments of the method in order to get a specific water-dispersible composition of interest.

A herein suitable solvent may propylene glycol - in working Example 1 herein was used propylene glycol as solvent.

Preferably is step (i) above made by hydrolyzing bixin under alkaline conditions (e.g. by use of KOH) in a suitable solvent (e.g. a solvent that includes propylene glycol) to produce the norbixin solution in a suitable solvent (e.g. a solvent that includes propylene glycol).

In working example 1 herein was made a norbixin solution in propylene glycol solvent by hydrolyzing bixin under alkaline conditions.

Without being limited to theory - it may be that norbixin is partly or completely esterified when e.g. an alcohol such as e.g. propylene glycol is used as solvent.

Without being limited to theory - quillaja as such could maybe be used as a suitable solvent.

Further, water at a suitable pH could also maybe be used as a suitable solvent.

Propylene glycol is liquid at room temperature - accordingly, if propylene glycol is used as a solvent one may directly get a liquid composition.

As discussed above - an example of a herein suitable quillaja product is a to 20% in water diluted quillaja product - if such water containing quillaja product is used one may directly get a liquid composition.

Further, if one e.g. wants a dry powder composition the method may comprise the further step of drying.

### A method for making a transparent orange/red solid-in-water dispersion

As discussed above, a method a method for making a transparent orange/red solid-in-water dispersion product of the invention is disclosed, wherein the method comprises dispersing the water-dispersible composition of into a water based product with a pH 1 to pH 8 to thereby obtain the transparent orange/red solid-in-water dispersion product of the invention.

In the present context may this method be seen as routine work for the skilled person and it may therefore not be described in great details herein.

As known to the skilled person in the present context - if the water-dispersible red composition as described herein is a relatively concentrated red composition then one may only need to add a few drops to a herein relevant water based product (e.g. a soft drink) in order to get a herein relevant suitable orange/red color of e.g. a soft drink.

### Use of the water-dispersible composition

As discussed above, the use of the water-dispersible composition for orange/red coloring of an edible, a drinkable or a pharmaceutical product is disclosed herein.

Essentially, this use may herein be seen as corresponding to known prior art uses of water-dispersible compositions comprising herein relevant coloring pigments.

Accordingly, it is herein not required in details to describe how such compositions are used for coloring of a herein relevant edible or pharmaceutical product - the skilled person knows how to use it in relation to obtainment of specific objectives (e.g. degree of coloring) of interest.

Preferably, the product is a food product or a beverage product - preferably for human use.

Preferred examples of edible or drinkable products may be a soft drink, wine gum, marmalade or jam.

### EXAMPLES

### EXAMPLE 1:

In the laboratory the following water-dispersible composition was produced:

| **Ingredient** | **Amount grams** | **% w/w on a water free basis** |
|---|---|---|
| Quillaja Q-Naturale® from National Starch, UK | 26 grams (20% pure - rest including water) => 5.2 grams Quillaja | Approximately 9% |
| Norbixin solution in propylene glycol | 52 grams => 50.4 grams water-free solution => approximately 3 grams norbixin and 47 grams propylene glycol | 90.6% => approximately |
| | | 5% norbixin |
| | | 85% propylene glycol |

The % sum (w/w on a water free basis) of Quillaja, norbixin and propylene glycol was approximately 99% - the rest of not water material was KOH and maybe some methanol from the hydrolysis of bixin.

The solution of norbixin in the solvent propylene glycol was prepared as described below:
4.4 grams of a 45% solution of KOH in demineralised water was added to 144 grams of propylene glycol and the solution was heated to 85°C. While heating and stirring 9.6 grams of crystalline bixin (90%) was added, the temperature was increased to 90°C and kept at 90°C until all bixin had dissolved and been hydrolyzed into norbixin. As soon as all bixin had dissolved the solution was cooled to room temperature and a norbixin solution in propylene glycol was obtained.
52 grams of the norbixin solution in propylene glycol was then added to 26 grams of the quillaja solution under stirring.

A dark orange-red liquid solution was obtained.

### EXAMPLE 2:

In the laboratory the product produced as described in Example 1 was tested as follows.

A few drops of the water water-dispersible orange/red composition of example 1 were added to a standard soft drink medium with pH of 3.

A nice transparent orange/red color was obtained.

The soft drink medium corresponds to a standard commercially available soft drink media.

The result was a transparent orange/red solid-in-water soft drink dispersion that was stable in the sense that it was still orange/red and transparent and showed no sedimentation after two days storage at room temperature.

### EXAMPLE 3:

This example is a comparative example using polyglycerol ester as a dispersing agent. In this example was used the same ingredients in approximately the same amounts as in Example 1 - except polyglycerol ester had replaced the quillaja - i.e. a true comparative example.

The comparative polyglycerol containing composition was tested as in Example 2.

The result was a soft drink dispersion that was not stable in the sense that after two days storage at room temperature there was a significant sedimentation of orange/red pigment - i.e. the soft drink had lost some of its orange/red color.

Accordingly, **the results of this comparative example clearly demonstrated that quillaja works significantly better than polyglycerol.**

### REFERENCES

1: Publicly available product information - natural red color product of Chr. Hansen A/S, Denmark termed "A-720-WS-AP"
2: Publicly available product information - natural red color product of Chr. Hansen A/S, Denmark is termed "CapColors®A-8-WSS-145".
3: WO2011/119290A1
4: WO2011/154407A1

## Claims

1. A transparent orange/red solid-in-water dispersion product comprising norbixin particles as an orange/red pigment and wherein the pH of the dispersion is from pH 1 to pH 8, **characterised in that** it contains quillaja as a dispersant agent and wherein the ratio (w/w) of norbixin and quillaja is a ratio from 0.01 to 10.

2. The transparent orange/red solid-in-water dispersion product of claim 1, wherein the ratio (w/w) of norbixin and quillaja is a ratio from 0.1 to 1.

3. The transparent orange/red solid-in-water dispersion product of any of claims 1 to 2, wherein the norbixin particles are having an average particle diameter of less than 1 µm.

4. The transparent orange/red solid-in-water dispersion product of any of claims 1 to 3, wherein the pH of the dispersion is from pH 1 to pH 5.

5. The transparent orange/red solid-in-water dispersion product of any of claims 1 to 4, wherein the dispersion product is a food product or a beverage product.

6. The transparent orange/red solid-in-water dispersion product of claim 5, wherein the product is a soft drink, wine gum, marmalade or jam.

## Patentansprüche

1. Transparentes orange/rotes Dispersionsprodukt mit Feststoff in Wasser mit Norbixinpartikeln als orange/rotem Pigment, wobei der pH-Wert der Dispersion pH 1 bis 8 ist, **gekennzeichnet dadurch, dass** es Quillaja als Dispersionsmittel enthält, und wobei das Verhältnis (Gew./Gew.) von Norbixin und Quillaja im Verhältnis von 0,01 bis 10 ist.

2. Transparentes orange/rotes Dispersionsprodukt mit Feststoff in Wasser nach Anspruch 1, wobei das Verhältnis (Gew./Gew.) von Norbixin und Quillaja von 0,1 bis 1 ist.

3. Transparentes orange/rotes Dispersionsprodukt mit Feststoff in Wasser nach einem der Ansprüche 1 bis 2, wobei die Norbixinpartikel einen durchschnittlichen Teilchendurchmesser von weniger als 1 µm haben.

4. Transparentes orange/rotes Dispersionsprodukt mit Feststoff in Wasser nach einem der Ansprüche 1 bis 3, wobei der pH-Wert der Dispersion pH 1 bis 5 ist.

5. Transparentes orange/rotes Dispersionsprodukt mit Feststoff in Wasser nach einem der Ansprüche 1 bis 4, wobei das Dispersionsprodukt ein Lebensmittel oder ein Getränk ist.

6. Transparentes orange/rotes Dispersionsprodukt mit Feststoff in Wasser nach Anspruch 5, wobei das Produkt ein alkoholfreies Getränk, Weingummi, Orangen- oder sonstige Marmelade ist.

## Revendications

1. Produit de dispersion solide-en-eau orange/rouge transparent comprenant des particules de norbixine comme pigment orange/rouge et où le pH de la dispersion est de pH 1 à pH 8, **caractérisé en ce qu'**il contient du Quillaja comme agent dispersant, et où le rapport (p/p) de norbixine et Quillaja est un rapport de 0,01 à 10.

2. Produit de dispersion solide-en-eau orange/rouge transparent selon la revendication 1, où le rapport (p/p) de norbixine et Quillaja est un rapport de 0,1 à 1.

3. Produit de dispersion solide-en-eau orange/rouge transparent selon l'une quelconque des revendications 1 à 2, où les particules de norbixine ont un diamètre de particule moyen inférieur à 1 µm.

4. Produit de dispersion solide-en-eau orange/rouge transparent selon l'une quelconque des revendications 1 à 3, où le pH de la dispersion est de pH 1 à pH 5.

5. Produit de dispersion solide-en-eau orange/rouge transparent selon l'une quelconque des revendications 1 à 4, où le produit de dispersion est un produit alimentaire ou un produit de boisson.

6. Produit de dispersion solide-en-eau orange/rouge transparent selon la revendication 5, où le produit est une boisson non alcoolisée, des gommes fruitées, de la marmelade ou confiture.
